# EUROPÄISCHE PATENTANMELDUNG

(11) **EP 0 733 623 A2**
(43) Veröffentlichungstag der Anmeldung: **25.09.1996**
(21) Anmeldenummer: 96104008.6
(22) Anmeldetag: 14.03.1996
(51) Int. Cl.: C07D 251/34

(54) **Verfahren zur Herstellung von 1,3,5-Tris-(2-hydroxy-alkyl)-isocyanuraten in Gegenwart organischer Phosphorverbindungen**

(30) Priorität: 22.03.1995 DE 19510325
(71) Anmelder: BASF AKTIENGESELLSCHAFT, 67056 Ludwigshafen (DE)
(72) Erfinder: Gutschoven, Frank, 67063 Ludwigshafen (DE); Becker, Rainer, Dr., 67098 Bad Dürkheim (DE); Melder, Johann-Peter, Dr., 67141 Neuhofen (DE); Van den Brande, Etienne, 2070 Kapellen (BE); Krader, Thomas, Dr., 2950 Kapellen (BE)

(57) **Zusammenfassung**

Verfahren zur Herstellung von 1,3,5-Tris-(2-hydroxyalkyl)-isocyanuraten aus Cyanursäure und aliphatischen Epoxiden, dadurch gekennzeichnet, daß man die Reaktion in Gegenwart katalytischer Mengen einer organischen Phosphorverbindung durchführt.

## Beschreibung

Die Erfindung betrifft ein Verfahren zur Herstellung von 1,3,5-Tris-(2-hydroxyalkyl)-isocyanuraten aus Cyanursäure und aliphatischen Epoxiden.

Es ist bekannt, daß man 1,3,5-Tris-(2-hydroxyalkyl)-isocyanurate im allgemeinen aus Cyanursäure und Epoxiden herstellt. (vgl. E. Amgwerd, Chemische Rundschau, Band 23, Nr. 45 aus 1970).

In der DE-B 1 670 214 wird empfohlen, diese Reaktion ohne Katalysator in einem niederen Alkohol oder einem wasserlöslichen Ether als Lösungsmittel durchzuführen.

Es wurde weiterhin vorgeschlagen, die Reaktion in Gegenwart von Basen wie Alkalien (vgl. US-A 3 088 948) als Katalysatoren vorzunehmen.

Ferner ist bekannt, als Katalysator Säuren wie Schwefel- oder Salzsäure (US-A 3 265 694 und 3 231 577) oder Alkali- oder Erdalkalisalze starker Mineralsäuren (JP-B 1970/15732) einzusetzen.

Die JP-B 1969/23322 beschreibt die Verwendung von Katalysatoren wie tertiäre Amine oder Derivate tertiärer Amine wie quaternisierte Amine.

Die vorstehend beschriebenen Verfahren weisen allerdings den Nachteil auf, daß die Reaktionsgeschwindigkeit auch bei erhöhter Temperatur noch zu langsam ist und man hohe Ausbeuten erst nach einigen Stunden Reaktionszeit erhält. Hinzu kommt, daß durch die damit verbundene thermische Belastung der Produkte und Ausgangsstoffe Nebenreaktionen begünstigt werden. Diese Folge tritt erst recht ein, wenn man zur Verkürzung der Zykluszeit die Reaktionstemperatur weiter erhöht.

Aufgabe der vorliegenden Erfindung war es deshalb, ein Verfahren zur Verfügung zu stellen, das bei niedrigen Temperaturen in hoher Raum-Zeit-Ausbeute die Herstellung von 1,3,5-Tris-(2-hydroxyalkyl)-isocyanuraten ermöglicht, ohne daß dabei wesentliche Mengen an Nebenprodukten gebildet werden.

Demgemäß wurde ein Verfahren zur Herstellung von 1,3,5-Tris-(2-hydroxyalkyl)-isocyanuraten aus Cyanursäure und aliphatischen Epoxiden gefunden, welches dadurch gekennzeichnet ist, daß man die Reaktion in Gegenwart katalytischer Mengen einer organischen Phosphorverbindung durchführt.

Das erfindungsgemäße Verfahren eignet sich insbesondere zur Herstellung von 1,3,5-Tris-(2-hydroxyalkyl)-isocyanuraten der allgemeinen Formel IV 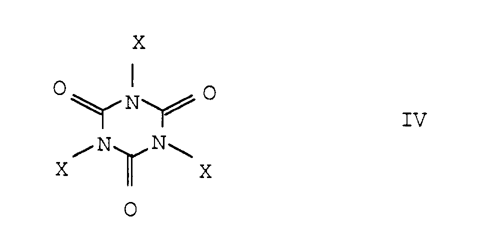 in der X ein Rest der allgemeinen Formel V 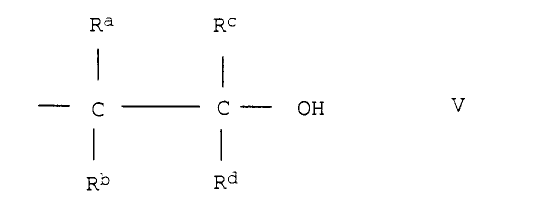 ist, und R^{a}, R^{b}, R^{c} und R^{d} Wasserstoff, C₁- bis C₁₀-Alkyl oder C₅- bis C₈-Cycloalkyl bedeuten. Wertvolle Zwischenprodukte sind vor allem 1,3,5-Tris-(2-hydroxyalkyl)-isocyanurate, bei denen die Reste R^{a}, R^{b} und R^{c} Wasserstoff und der Rest R^{d} ein C₁- bis C₁₀-Alkyl oder C₅- bis C₈-Cycloalkyl bedeuten.

Von besonderer wirtschaftlicher Bedeutung sind 1,3,5-Tris-(2-hydroxyethyl)-isocyanurat, 1,3,5-Tris-(2-hydroxypropyl)-isocyanurat.

Die Herstellung der gewünschten 1,3,5-Tris-(2-hydroxyalkyl)-isocyanurate erfolgt durch Umsetzung von Cyanursäure mit den entsprechenden Epoxiden.

Als aliphatische Epoxide eignen sich insbesondere solche der allgemeinen Formel I 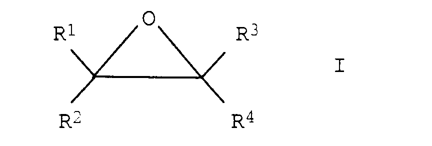 in der R¹, R², R³ und R⁴ Wasserstoff, C₁- bis C₁₀-Alkyl oder C₅- bis C₈-Cycloalkyl bedeuten. Insbesondere kommen preisgünstige aliphatische Epoxide wie Ethylenoxid, Propylenoxid, Butylenoxid und Isobutylenoxid in Betracht.

Bei der Umsetzung hat es sich bewährt, das aliphatische Epoxid in einem molaren Überschuß bezogen auf die Menge der Stickstoffatome in der Cyanursäure einzusetzen. Das Molverhältnis aliphatisches Epoxid zu Cyanursäure beträgt deshalb mindestens 3 : 1 , bevorzugt 3 : 1 bis 3,8 : 1, besonders bevorzugt 3,1 : 1 bis 3,6 : 1.

Die Reaktion wird in Gegenwart von organischen Phosphorverbindungen wie Phosphoranen, Phosphiten, Phospholidinen und besonders günstig mit Phosphinen oder Aminophosphine durchgeführt.

Geeignete Phosphine sind beispielsweise solche der allgemeinen Formel II 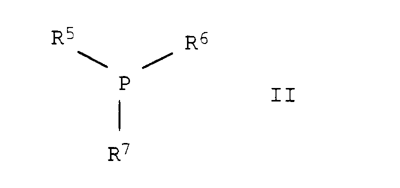 in der R⁵, R⁶ und R⁷ die folgende Bedeutung haben:
Wasserstoff, C₁- bis c₂₀-, insbesondere C₁- bis C₆- Alkyl, C₃- bis C₈-, insbesondere C₅- und C₆-Cycloalkyl, C₆- bis C₂₀-Aryl, insbesondere Phenyl, C₅- bis C₂₀-Heteroaryl, C₇- bis C₂₀-Alkylaryl, insbesondere Toluyl, C₇- bis C₂₀-Arylalkyl, insbesondere Benzyl oder ein Rest der allgemeinen Formel III 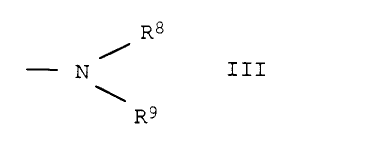 in der R⁸ und R⁹ Wasserstoff, C₁- bis C₂₀-, insbesondere C₁- bis C₆- Alkyl, C₃- bis C₈-, insbesondere C₅- und C₆-Cycloalkyl, C₆- bis C₂₀-Aryl, insbesondere Phenyl, C₅- bis C₂₀-Heteroaryl, C₇- bis C₂₀-Alkylaryl, insbesondere Toluyl, C₇- bis C₂₀-Arylalkyl, insbesondere Benzyl bedeuten.

Unter den handelsüblichen Phosphinen sind besonders Triphenylphosphin, Tris-(diethylamino)-phosphin oder Tris-(dimethylamino)-phosphin geeignet.

Die organischen Phosphorverbindungen sind bereits in einer Menge von 500 mol-ppm organische Phosphorverbindung, bezogen auf die Cyanursäure, wirksam. Üblicherweise setzt man bis zu 50 000 mol-ppm, bezogen auf die Cyanursäure, ein. Besonders gute Ergebnisse erzielt man, wenn man sie in Mengen von 2000 bis 10000 mol-ppm, bezogen auf die Cyanursäure, einsetzt.

Vorteilhaft wird in katalytischen Mengen neben den organischen Phosphorverbindungen als Co-Katalysator ein tertiäres Amin, ein Ammoniumsalz eines tertiären Amins oder eine quartäre Ammoniumverbindung mitverwendet.

Geeignet sind Amine mit einer oder mehreren tertiären Aminogruppen, die organische Reste wie C₁- bis C₂₀-, insbesondere C₁- bis C₆- Alkyl, C₃- bis C₈-, insbesondere C₅- und C₆-Cycloalkyl, C₆- bis C₂₀-Aryl, insbesondere Phenyl, C₅- bis C₂₀-Heteroaryl, C₇- bis C₂₀-Alkylaryl, insbesondere Toluyl, C₇- bis C₂₀-Arylalkyl, insbesondere Benzyl tragen. Bei den C₁- bis C₂₀-Alkyl- und den C₃- bis C₈- Cycloalkylgruppen können auch ein oder 2 Wasserstoffatome durch Hydroxylgruppen ersetzt sein. Unter den tertiären Aminen mit mehreren tertiären Aminogruppen kommen insbesondere solche in Betracht, bei denen die Stickstoffatome mit Einheiten miteinander verbunden sind, die sich durch Abstraktion von 2 Wasserstoffatomen von C₁- bis C₁₀-Kohlenwasserstoffverbindungen ableiten, z.B. Einheiten wie Ethylen, Propandiyl, Butandiyl oder Phenylen.

Konkrete Beispiele für geeignete tertiäre Amine sind Triethylamin, Triethanolamin und 1,4-Diazabicyclo[2.2.2]octan.

Die tertiären Amine können auch in Form ihrer Ammoniumsalze eingesetzt werden. Dabei kommen insbesondere die Addukte aus starken organischen Säuren und Mineralsäuren, z.B. Halogenwasserstoffsäuren oder Schwefelsäure an die tertiären Amine in Betracht.

Als organische Reste der quartären Ammoniumverbindungen kommen die gleichen in Betracht, die als organische Reste der tertiären Amine genannt sind.

Als Gegenionen der quartären Ammoniumionen kommen neben dem Hydroxylion die Anionen starker organischer Säuren oder Mineralsäuren wie der Halogenwasserstoffsäuren, der Schwefelsäure und der Phosphorsäure in Betracht.

Beispiele für geeignete quartäre Ammoniumverbindungen sind Benzyl-trimethylammoniumhydroxid, Tetrakis-(2-hydroxyethyl)-ammoniumhydroxid oder Benzyl-dimethyl-2-hydroxyethyl-ammoniumhydroxid.

Im allgemeinen setzt man pro mol organische Phosphorverbindung 0,05 bis 20, bevorzugt 0,2 bis 5 mol von dem Co-Katalysator ein.

Die Ausgangsstoffe werden im allgemeinen bei Temperaturen von 0 bis 180°C zur Reaktion gebracht. Gut verläuft die Reaktion bei 100 bis 140°C; besonders gut verläuft die Reaktion bei 110 bis 130°C.

Die Reaktion kann bei normalem Druck durchgeführt werden. Ein höherer Druck als Normaldruck empfiehlt sich beim Einsatz von aliphatischen Epoxiden, die bei den bevorzugten Reaktionstemperaturen unter Normaldruck gasförmig sind. Beim Einsatz von Ethylenoxid und Propylenoxid wendet man deshalb mit Vorteil Drücke von 3 bis 20 bar an.

Bevorzugt erfolgt die Umsetzung der Ausgangsstoffe nach dem erfindungsgemäßen Verfahren in Gegenwart von allgemein üblichen Lösungsmitteln, die mit den aliphatischen Epoxiden und der Cyanursäure nur langsam reagieren. Bevorzugt sind niedere Alkohole mit 1 bis 6 Kohlenstoffatomen, insbesondere Propanol und Isopropanol, niedere Ether mit 2 bis 6 Kohlenstoffatomen, z. B. Tetrahydrofuran oder Dioxan, Ketone mit 3 bis 6 Kohlenstoffatomen, z.B. Aceton oder Butanon, Amide wie Dimethylformamid oder Dimethylacetamid.

Im allgemeinen setzt man die Lösungsmittel in einer Menge von 10 bis 1000 Gew.-%, bezogen auf die Reaktanden, ein. Die Menge wird günstigerweise so gewählt, daß sie ausreicht, um die Ausgangsstoffe bei den bevorzugten Reaktionstemperaturen zu lösen.

Das erfindungsgemäße Verfahren führt man beispielsweise aus, indem man zu einer Lösung aus Cyanursäure, der organischen Phosphorverbindung und gegebenenfalls dem Co-Katalysator und einem geeigneten Lösungsmittel die aliphatischen Epoxide bei den angegebenen Temperaturen im beschriebenen Verhältnis zudosiert und bei dieser Temperatur reagieren läßt, bis die Cyanursäure nahezu vollständig zum 1,3,5-Tris-(2-hydroxyalkyl)-isocyanurat abreagiert ist. Der erreichte Umsatz läßt sich mit üblichen analytischen Methoden (z.B. Infrarotspektroskopie) überwachen.

Die Isolierung des 1,3,5-Tris-(2-hydroxyalkyl)-isocyanurates erfolgt nach üblichen, dem Fachmann bekannten Methoden, z.B. durch Abdestillieren des Lösungsmittels und der uberschüssigen aliphatischen Epoxide oder Kristallisation des 1,3,5-Tris-(2-hydroxyalkyl)-isocyanurates bei niedriger Temperatur und anschließendes Abfiltrieren.

Im allgemeinen reichen Reaktionszeiten von 1 bis 36, bevorzugt 3 bis 6 h aus, um das gewünschte 1,3,5-Tris-(2-hydroxyalkyl)-isocyanurat in hoher Reinheit und Ausbeute zu erhalten.

### Beispiele 1 bis 5

In einem Reaktor wurden 97 g (0,75 mol) Cyanursäure, 0,0034 mol einer organischen Phosphorverbindung und gegebenenfalls (Beispiele 4 und 5) 0,0034 mol eines tertiären Amins und 400 ml n-Propanol vorgelegt. Bei einer Reaktionstemperatur von 120°C wurde über 3 h hinweg kontinuierlich mit bei allen Versuchen gleicher Geschwindigkeit insgesamt 51 g (1,2 mol) Ethylenoxid eingeleitet.

Nach Ablauf der 3 Stunden wurde der Reaktorinnendruck gemessen und der Reaktor anschließend auf Raumtemperatur abgekühlt. Die Reaktionsmischung enthielt neben unumgesetztem Ethylenoxid, unumgesetzte Cyanursäure, Monohydroxyethylisocyanurat, Dihydroxyethylisocyanurat das gewünschte 1,3,5-Tris(-2-hydroxyethyl)isocyanurat als Hauptprodukt.

Da sichergestellt war, daß während der Umsetzung das Volumen des Reaktors konstant und ein Entweichen von Materie aus dem Reaktor unmöglich war, konnte die Reaktionsgeschwindigkeit indirekt über den Reaktorinnendruck am Ende der Reaktionszeit (3 h) ermittelt werden. Je höher der Reaktorinnendruck, desto geringer der Umsatz an Ethylenoxid, und desto geringer war folglich die Reaktionsgeschwindigkeit.

### Beispiele 6 und 7 (zum Vergleich)

Die Versuche wurden analog zu denen der Beispiele 1 bis 5 durchgeführt, jedoch wurden statt 0,0034 mol einer organischen Phosphorverbindung 0,0034 mol eines tertiären Amins eingesetzt.

Die Versuchsergebnisse können der Tabelle 1 entnommen werden.

**Tabelle 1**

| Beispiel | Katalysator | Reaktorinnendruck [bar] |
|---|---|---|
| 1 | Triphenylphosphin | 7,9 |
| 2 | Tris-(diethylamino)-phosphin | 7,7 |
| 3 | Tris-(dimethylamino)-phosphin | 7,6 |
| 4 | Triphenylphosphin/Triethylamin | 7,4 |
| 5 | Triphenylphosphin/Triethanolamin | 7,8 |
| 6* | Triethanolamin | 8,5 |
| 7* | Triethylamin | 8,0 |

| | | |
|---|---|---|
| *zum Vergleich | | |

### Beispiele 8 bis 11

In einem Reaktor wurden 97 g (0,75 mol) Cyanursäure, eine organischen Phosphorverbindung und gegebenenfalls (Beispiele 9 und 10) eines tertiären Amins und 400 ml n-Propanol vorgelegt. Bei einer Reaktionstemperatur von 120°C wurden innerhalb von 0,5 h 17 g (0,39 mol) Ethylenoxid eingeleitet, wobei der Reaktorinnendruck auf 8,1 bar anstieg. Danach wurde bei der angegebenen Temperatur insgesamt 89 g (2,02 mol) Ethylenoxid nachgepreßt, wobei dies nur in dem Umfang erfolgt, daß der Druck konstant 8,1 bar betrug. Die Zeit, die erforderlich ist, um diese Menge an Ethylenoxid nachzupressen, ist ein Maß für die Reaktionsgeschwindigkeit und kann, neben anderen Verfahrensparametern, Tabelle 2 entnommen werden.

Die eingesetzte Menge an Phosphorverbindung und Aminoverbindung betrug bei den Beispielen 8, 9 und 11 jeweils 0,0034 mol. Bei Beispiel 10 betrugen die entsprechenden Mengen 0,0017 mol.

### Beispiel 11 (zum Vergleich)

Beispiel 8 wurde wiederholt, wobei statt Triphenylphosphin 0,52 g (0,0034 mol) Triethanolamin eingesetzt wurde.

**Tabelle 2**

| Beispiel | Katalysator | Nachpreßzeit [h] | Ausbeute ¹⁾[%] |
|---|---|---|---|
| 8 | Triphenylphosphin | 2,6 | 89 |
| 9 | Triphenylphosphin/Triethylamin | 3,25 | 88 |
| 10 | Triphenylphosphin/Triethylamin | 4,1 | |
| 11* | Triethanolamin | 5,6 | 87 |

| | | | |
|---|---|---|---|
| *zum Vergleich | | | |
| ¹⁾ an Tris(hydroxyethyl)isocyanurat | | | |

### Beispiel 12

In einem Reaktor wurden 97 g (0,75 mol) Cyanursäure, 0,55 g (0,0034 mol) Tris-(dimethylamino)phosphin und 400 ml Dimethylformamid vorgelegt. Anschließend wurde soviel Stickstoff aufgepreßt, daß der Innendruck bei Raumtemperatur 3 bar betrug. Anschließend wurde die Reaktionsmischung auf 120°C aufgeheizt. Bei dieser Temperatur wurden über 3 h hinweg insgesamt 51 g (1,2 mol) Ethylenoxid mit konstanter Dosiergeschwindigkeit aufgepreßt. Der Innendruck betrug nach 3 h 0,5 bar. Die Höhe des Innendrucks ist ein Maß für die Reaktionsgeschwindigkeit, wobei hoher Druck ein geringer Umsatz an Ethylenoxid und somit eine geringe Reaktionsgeschwindigkeit bedeutet.

### Beispiel 13

Beispiel 12 wurde wiederholt, wobei jedoch 0,19 g (0,0034 mol) KOH (Kaliumhydroxid) als Katalysator eingesetzt wurde. Der Innendruck betrug nach 3stündigem Einleiten von Ethylenoxid 0,7 bar.

### Beispiel 14

In einem Reaktor wurden 97 g (0,75 mol) Cyanursäure, 900 mg Triphenylphosphin und 400 ml n-Propanol vorgelegt. Bei einer Reaktionstemperatur von 120°C wurde über 17 h hinweg insgesamt 107 g (1,8 mol) Propylenoxid eingeleitet. Anschließend wurde die Reaktion durch Abkühlen abgebrochen, der Reaktor entspannt und der Reaktionsaustrag analysiert. An 99,8 mol-% der Cyanursäure war wenigstens eine Propylenoxideinheit addiert worden.

## Patentansprüche

1. Verfahren zur Herstellung von 1,3,5-Tris-(2-hydroxyalkyl)-isocyanuraten aus Cyanursäure und aliphatischen Epoxiden, dadurch gekennzeichnet, daß man die Reaktion in Gegenwart katalytischer Mengen einer organischen Phosphorverbindung durchführt.

2. Verfahren nach Anspruch 1, dadurch gekennzeichnet, daß man als aliphatisches Epoxid eine Verbindung der allgemeinen Formel I 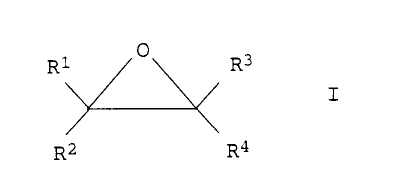 einsetzt, in der R¹, R², R³ und R⁴ Wasserstoff, C₁- bis C₁₀-Alkyl oder C₅- bis C₈-Cycloalkyl bedeuten.

3. Verfahren nach Anspruch 1 oder 2, dadurch gekennzeichnet, daß man als organische Phosphorverbindung ein Phosphin der allgemeinen Formel II 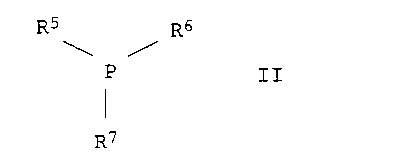 in der R⁵, R⁶ und R⁷ die folgende Bedeutung haben:
Wasserstoff, C₁- bis C₂₀- Alkyl, C₃- bis C₈-Cycloalkyl, C₆- bis C₂₀-Aryl, C₅- bis C₂₀-Heteroaryl, C₇- bis C₂₀-Alkylaryl, C₇- bis C₂₀-Arylalkyl oder ein Rest der allgemeinen Formel III 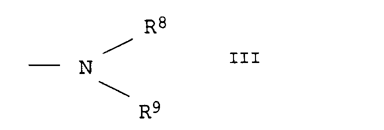 in der R⁸ und R⁹ Wasserstoff, C₁- bis C₂₀- Alkyl, C₃- bis C₈-Cycloalkyl, C₆- bis C₂₀-Aryl, C₅- bis C₂₀-Heteroaryl, C₇- bis C₂₀-Alkylaryl oder C₇- bis C₂₀-Arylalkyl bedeuten,
einsetzt.

4. Verfahren nach den Ansprüchen 1 bis 3, dadurch gekennzeichnet, daß man 500 bis 50000 mol-ppm organische Phosphorverbindung, bezogen auf die Cyanursäure, einsetzt.

5. Verfahren nach den Ansprüchen 1 bis 4, dadurch gekennzeichnet, daß man die Reaktion bei einer Temperatur von 100 bis 140°C durchführt.

6. Verfahren nach den Ansprüchen 3 bis 5, dadurch gekennzeichnet, daß man Triphenylphosphin, Tris-(diethylamino)-phosphin oder Tris-(dimethylamino)-phosphin einsetzt.

7. Verfahren nach den Ansprüchen 1 bis 6, dadurch gekennzeichnet, daß man die Reaktion in Gegenwart katalytischer Mengen eines Co-Katalysators, ausgewählt aus der Gruppe der tertiären Amine, der Ammoniumsalze tertiärer Amine und der quartären Ammoniumverbindungen durchführt.

8. Verfahren nach Anspruch 7, dadurch gekennzeichnet, daß man als Co-Katalysator Triethylamin, 1,4-Diazabicyclo[2.2.2]octan, Benzyl-trimethylammoniumhydroxid, Tetrakis-(2-hydroxyethyl)-ammnoniumhydroxid, Triethanolamin oder Benzyl-dimethyl-2-hydroxyethyl-ammoniumhydroxid einsetzt.

9. Verfahren nach Anspruch 7 oder 8, dadurch gekennzeichnet, daß man pro Mol organische Phosphorverbindung 0,05 bis 20 mol von dem Co-Katalysator einsetzt.

10. Verfahren nach den Ansprüchen 1 bis 9, dadurch gekennzeichnet, daß man die Reaktion in Lösung mit Propanol oder Dimethylformamid als Lösungsmittel durchführt.
